# EUROPEAN PATENT SPECIFICATION

(11) **EP 3 956 298 B1**
(45) Date of publication and mention of the grant of the patent: **04.11.2026**
(21) Application number: 20720039.5
(22) Date of filing: 17.04.2020
(51) Int. Cl.: C07C 253/30, C07C 255/16

(54) **PROCESS FOR PREPARING CYANOACETATES**
VERFAHREN ZUR HERSTELLUNG VON CYANOACETATEN
PROCÉDÉ DE PRÉPARATION DE CYANOACÉTATES

(30) Priority: 18.04.2019 GB 201905553
(43) Date of publication of application: 23.02.2022
(73) Proprietor: Henkel AG & Co. KGaA, 40589 Düsseldorf (DE)
(72) Inventor: DUFFY, Cormac, Jenkinstown Dundalk, Co. Louth (IE); O'SULLIVAN, Justine, Boston Rathangan, Co. Kildare R51 EH98 (IE); GOFF, Ciara, Enniscorthy, Co. Wexford (IE); FARID, Umar, Dublin, Co. Dublin D02 CC84 (IE); RAMOS, Jessica, Maynooth, Co. Kildare W23 P9T2 (IE); THAI TRUNG KING, Michael, Clarehall, Co. Dublin D13 (IE); COBO CARDENETE, Isidro, Co. Dublin D12 (IE); PHELAN, Marisa, Co, Dublin D9 (IE); BURNS, Barry, Co. Dublin (IE)
(86) International application number: PCT/EP2020/060882
(87) International publication number: WO 2020/212583

(56) References cited:
- L. SPIEGEL: "Über die direkte Umwandlung von Nitrilen in Ester. Nach Versuchen von H. Szydlowsky", BERICHTE DER DEUTSCHEN CHEMISCHEN GESELLSCHAFT, vol. 51, 1918, WILEY-VCH Verlag, Weinheim, DE, pages 296 - 298, XP055706700, ISSN: 0009-2940, DOI: 10.1002/cber.19180510134
- W.J. GREENLEE, ET AL.: "Mild conversion of carboxamides and carboxylic acid hydrazides to acids and esters", JOURNAL OF ORGANIC CHEMISTRY, vol. 46, no. 25, 1 December 1981 (1981-12-01), American Chemical Society, Washington, DC, US, pages 5351 - 5353, XP002124735, ISSN: 0022-3263, DOI: 10.1021/JO00339A019
- ZHONG-LIU WU, ET AL.: "Enantioselective biotransformation of alpha,alpha-disubstituted dinitriles to the corresponding 2-cyanoacetamides using Rhodococcus sp. CGMCC 0497", TETRAHEDRON: ASYMMETRY, vol. 14, no. 15, 18 July 2003 (2003-07-18), Elsevier Science Publishers, Oxford, GB, pages 2133 - 2142, XP004441347, ISSN: 0957-4166, DOI: 10.1016/S0957-4166(03)00456-7

## Description

### BACKGROUND

### Field

This invention relates to a process for producing cyanoacetates using a cyanoacetamide as a precursor.

### Brief Description of Related Technology

Cyanoacrylate adhesives are known for their fast adhesion and ability to bond a wide variety of substrates. They are marketed as "super glue" type adhesives. They are useful as an all-purpose adhesive since they are a single component adhesive, very economical as only a small amount will do, and generally do not require any equipment to effectuate curing.

Traditionally, cyanoacrylate monomers have been produced by way of a Knoevenagel condensation reaction between a formaldehyde precursor, such as paraformaldehyde, and an alkyl cyanoacetate with a basic catalyst. During the reaction, cyanoacrylate monomer forms and polymerises *in situ* to a prepolymer. The prepolymer is subsequently thermally cracked or depolymerised, yielding cyanoacrylate monomer. This approach has remained essentially the same over time, though various improvements and variants have been introduced. See e.g. U.S. Patent Nos. 6,245,933, 5,624,699, 4,364,876, 2,721,858, 2,763,677 and 2,756,251. Thus, it is seen one use of cyanoacetates is in the formation of cyanoacrylates.

Vijayalakshmi et al., J. Ad. Sci. Technol., 4, 9, 733 (1990) describes some approaches to the synthesis of cyanoacetates and corresponding cyanoacrylates, including preparation from chloroacetic acid and its esters by subsequent reaction with sodium cyanide.

Guseva et al., Russia Chem. Bull., 42, 3, 478 (1993) describes functionalized cyanoacetates, many of which were used in the subsequent synthesis of corresponding cyanoacrylates. [See also Guseva et al., Russia Chem. Bull., 43, 4, 595 (1994), and Golobolov and Gruber, Russia Chem. Rev., 66, 11, 953 (1997).] Cyanoacetates with siliconised functionalities have been described. See e.g. Senchenya et al., Russia Chem. Bull., 42, 5, 909 (1993) and European Patent Document No. EP 0 459 617.

The preparation of mono-, di-, tri- and tetrafunctional cyanoacetates, albeit as curatives for epoxy resins for adhesive applications, has been described. Renner et al., "Cure of Epoxy Resins with Esters of Cyanoacrylic Acid", J. Polym. Sci., Polym. Chem. Ed., 23, 2341 (1985) and U.S. Patent Nos. 4,202,920 and 4,517,357.

Greenlee et al, J. Org. Chem., 46, 26, 5351 (1981) describes the preparation of methyl cyanoacetate by the reaction of cyanoacetamide with Amberlyst 15 resin in methanol.

Notwithstanding the state of the technology it would be desirable to find alternative synthetic approaches to making cyanoacetates, particularly if such approaches used readily available and inexpensive starting materials. It would be even more desirable if such approaches generated the subject cyanoacetate in high yield, was readily isolated, and used at least starting materials that are recognized as being safe.

### SUMMARY

At a high level, the inventive process provides for the preparation of a cyanoacetate, steps of which comprise:
(a) contacting a cyanoacetamide with an alcohol, in the presence of a mineral acid, under appropriate conditions and for a time sufficient to yield a cyanoacetate; and,
(b) optionally, separating therefrom the so formed cyanoacetate,
wherein said cyanoacetamide is selected from cyanoacetamide, mono-substituted cyanoacetamides or disubstituted cyanoacetamides of which any substituents are alkyl, alkenyl, alkynyl or aryl.

The separation step should yield the cyanoacetate substantially free from the cyanoacetamide, the alcohol, mineral acid, and by-products.

### DETAILED DESCRIPTION

As noted above, the present invention provides a process for the preparation of a cyanoacetate, steps of which comprise:
(a) contacting a cyanoacetamide with an alcohol, in the presence of a mineral acid, under appropriate conditions and for a time sufficient to yield a cyanoacetate; and,
(b) optionally, separating therefrom the so formed cyanoacetate,
wherein said cyanoacetamide is selected from cyanoacetamide, mono-substituted cyanoacetamides or disubstituted cyanoacetamides of which any substituents are alkyl, alkenyl, alkynyl or aryl.
The separation step should yield the cyanoacetate substantially free from the cyanoacetamide, the alcohol, mineral acid, and by-products.

The cyanoacetate formed by the inventive process may be a C₁₋₂₀ alkyl cyanoacetate, a C₆₋₂₀ aryl cyanoacetate, a C₇₋₂₀ alkaryl cyanoacetate or a C₇₋₂₀ aralkyl cyanoacetate, any of which may be substituted by one or more hydroxyl groups or C₁₋₂₀ alkyl ether groups.

More specifically, the cyanoacetate may be a C₁₋₂₀ alkyl cyanoacetate, where the C₁₋₂₀ alkyl may be straight chain or branched, contain one or more points of unsaturation and may be substituted and/or interrupted by one or more heteroatoms or heteroatom-containing groups (such as trimethylsilyl alkyl, like methyl, ethyl or propyl), or substituted by halogens or substituted or interrupted by halogen-containing groups. For instance, the cyanoacetate may be methyl, ethyl, propyls (like n-propyl or iso-propyl), propargyl, butyls (like n-butyl or iso-butyl), pentyls (like n-pentyl or iso-amyl), hexyl, octyls (like n-octyl or 2-ethylhexyl), nonyl, oxononyl, decyl, dodecyl, allyl, ethynyl, butenyl, cyclohexyl, tetrahydrofurfuryl, chloroethyl, 2,2,2-trifluoroethyl, hexafluoroisopropyl, alkoxy ether alkyl cyanoacetates (such as methoxymethyl, methoxyethyl, methoxybutyl, ethoxyethyl, propoxyethyl, butoxymethyl, or butoxyethyl) and dimethyl siloxane esters of 2-cyanoacetic acid. This recitation is by no means however exhaustive.

Desirably, the so formed cyanoacetate should be a C₁₋₂₀ alkyl cyanoacetate selected from methyl cyanoacetate, ethyl cyanoacetate, propyl cyanoacetates, butyl cyanoacetates, pentyl cyanoacetates, octyl cyanoacetates, alkoxy ether alkyl cyanoacetates, allyl cyanoacetates, and combinations thereof.

The cyanoacetate may also be a C₆₋₂₀ aryl cyanoacetate such as phenyl cyanoacetate.

Or, the cyanoacetate may be a C₇₋₂₀ aralkyl cyanoacetate such as phenethyl cyanoacetate, benzyl cyanoacetate, and toluyl cyanoacetate, to name a few.

In conducting the process, a cyanoacetamide is the starting material or precursor to the cyanoacetate.

The cyanoacetamide used in the process of the invention is selected from cyanoacetamide, mono- or di-substituted cyanoacetamides, where the substitutions may be alkyl, alkenyl, alkynyl or aryl. For instance, alkyl substituted cyanoacetamides include methyl, ethyl, propyl, or butyl substituted cyanoacetamides; aryl substituted cyanoacetamides include phenyl or benzyl cyanoacetamides. Commercially available examples of such cyanoacetamide derivatives include N,N-diethyl-2-cyanoacetamide, for instance.

The cyanoacetamide should be used in an amount of about 1 equivalent against which the other reactants may be measured. The term "equivalent" is intended to capture molar equivalent, whenever it is used herein.

An alcohol is used to perform the esterification of step (a). The alcohol chosen may be an alkyl alcohol, an aryl alcohol, an alkaryl alcohol or an aralkyl alcohol. The identity of the chosen alcohol depends on the desired cyanoacetate sought to be prepared. Accordingly, the alcohol may be selected from methanol, ethanol, propanols (such as isopropanol), proparganols, butanols (such as isobutanol), pentanols (such as isoamyl alcohol), hexanols, octanols, nonanols, oxononanols, decanols, dodecanols, allanol, cyclohexanol, tetrahydrofurfurol, chloroethanol, 2,2,2-trifluoroethanol, hexafluoroisopropanol, alkoxy ether alkanols (such as methoxymethanol, methoxyethanol, methoxybutanol, ethoxyethanol, propoxyethanol, butoxymethanol, or butoxyethanol), dialkyl siloxanols (such as dimethyl siloxanol or diethyl siloxanol), trialkylsilylalkanols (such as trimethylsilylmethanol, trimethylsilylethanol or trimethylsilylpropanol), should the corresponding respective alkyl cyanoacetate ester sought to be produced. Or, if the chosen alcohol is an aromatic alcohol, such as phenol, benzyl alcohol, then the corresponding aryl cyanoacetate ester would be the desired product.

The alcohol should be used in an amount of about 2.5 to about 25 equivalents, such as about 5 to about 10 equivalents, desirably about 5 to about 7.5 equivalents.

The mineral acid used in the inventive process may be selected from sulfuric acid, sulfurous acid, sulfonic acid, phosphoric acid, phosphorous acid, phosphonic acid, hydrochloric acid or hydrobromic acid.

The mineral acid should be used in an amount of about 0.5 to about 1.5 equivalents to about 1 equivalent of a cyanoacetamide, such as in an amount of about 0.6 to about 1.2 equivalents to about 1 equivalent of the cyanoacetamide, desirably about 0.6, about 0.9 or about 1.2 equivalents to about 1 equivalent of the cyanoacetamide starting material.

The alcohol should be used in excess to either or both of the cyanoacetamide and the mineral acid.

In the inventive process, the cyanoacetate is formed in a yield of about 70% or greater, such as about 90% or greater.

While the time of reaction is generally given above, the time may be monitored by reference to the formation of the desired product using NMR spectrometry, as noted in the Examples. The time of reaction may be adjusted depending on the identity of the specific reactants, the scale of the reaction and whether heat is added to the reaction conditions.

For the optional step of (b) appropriate isolation and/or separation techniques may be used to obtain the cyanoacetate.

The following examples are intended to illustrate but in no way limit the present invention.

### EXAMPLES

We used reaction conditions reported in Z.-L. Wu et al., Tetrahedron: Asymmetry, 14, 2133-42 (2003) to esterify the amide of a cyanoacetamide, along the synthetic scheme set forth below:

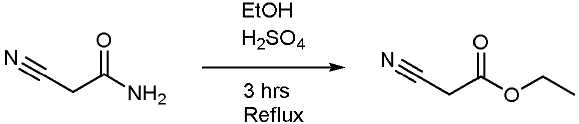

Applying the synthesis conditions of Z.-L. Wu the ethyl ester of cyanoacetate was obtained. The yields varied, as shown below in Table. The sulfuric acid equivalents were varied as was the time of reaction in the last instance. Table 1 below shows each of the six entries, all having 1 equivalent of the cyanoacetamide (30 grams) and 5.75 equivalents of ethanol (94.5 grams).

**Table 1**

| **Entry** | **H₂SO₄ (eq)** | **Time (hrs)** | **Yield (%)** |
|---|---|---|---|
| 1 | 0.3 | 3 | 41 |
| 2 | 0.15 | 3 | 12 |
| 3 | 0.6 | 3 | 69 |
| 4 | 1.2 | 3 | 94 |
| 5 | 0.9 | 3 | 97 |
| 6 | 0.3 | 17 | 32 |

From Entries 3-5, it may be seen that only those having about 70% or higher are within the scope of the inventive process. Thus, the mineral acid range of about 0.6 to about 1.2 is seen to be significant in order to obtain the desired yields.

Confirmation of formation of the ethyl cyanoacetate was obtained by NMR spectral analyses.

## Claims

1. A process for the preparation of a cyanoacetate, steps of which comprise:
(a) contacting a cyanoacetamide with an alcohol, in the presence of a mineral acid, under appropriate conditions and for a time sufficient to yield a cyanoacetate; and,
(b) optionally, separating therefrom the so-formed cyanoacetate,
wherein said cyanoacetamide is selected from cyanoacetamide, mono-substituted cyanoacetamides or disubstituted cyanoacetamides of which any substituents are alkyl, alkenyl, alkynyl or aryl.

2. The process of Claim 1, wherein the cyanoacetate is a C₁₋₂₀ alkyl cyanoacetate, a C₆₋₂₀ aryl cyanoacetate, a C₇₋₂₀ alkaryl cyanoacetate or a C ₇₋₂₀ aralkyl cyanoacetate, any of which may be substituted by one or more hydroxyl groups or C₁₋₂₀ alkyl ether groups.

3. The process of Claim 1, wherein the cyanoacetate is a C₁₋₂₀ alkyl cyanoacetate, wherein the C₁₋₂₀ alkyl may contain one or more points of unsaturation and may be substituted and/or interrupted by one or more heteroatoms or heteroatom-containing groups, or substituted by halogens or substituted or interrupted by halogen-containing groups.

4. The process of Claim 1, wherein the cyanoacetate is a C₁₋₂₀ alkyl cyanoacetate selected from methyl cyanoacetate, ethyl cyanoacetate, propyl cyanoacetates, butyl cyanoacetates, pentyl cyanoacetates, octyl cyanoacetates, alkoxy ether alkyl cyanoacetates, allyl cyanoacetate, and combinations thereof.

5. The process of Claim 1, wherein the cyanoacetate is a C₆₋₂₀ aryl cyanoacetate selected from phenyl cyanoacetate.

6. The process of Claim 1, wherein the cyanoacetate is a C₇₋₂₀ aralkyl cyanoacetate selected from phenethyl cyanoacetate, benzyl cyanoacetate, or toluyl cyanoacetate.

7. The process of Claim 1, wherein the alcohol is an alkyl alcohol, an aryl alcohol, an alkaryl alcohol or an aralkyl alcohol.

8. The process of Claim 1, wherein the alcohol is selected from methanol, ethanol, propanols, proparganols, butanols, pentanols, hexanols, octanols, nonanols, oxononanols, decanols, dodecanols, allanol, cyclohexanol, tetrahydrofurfurol, chloroethanol, 2,2,2-trifluoroethanol, hexafluoroisopropanol, alkoxy ether alkanols, dialkyl siloxanols, or trialkylsilylalkanols.

9. The process of Claim 1, wherein the alcohol is an aromatic alcohol,
preferably wherein the alcohol is selected from phenol or benzyl alcohol.

10. The process of Claim 1, wherein the mineral acid is sulfuric acid, sulfurous acid, sulfonic acid, phosphoric acid, phosphorous acid, phosphonic acid, hydrochloric acid or hydrobromic acid.

11. The process of Claim 1, wherein the mineral acid is used in an amount of 0.5 to 1.5 equivalents to 1 equivalent of the cyanoacetamide.

12. The process of Claim 1, wherein the mineral acid is used in an amount of 0.6 to 1.2 equivalents to 1 equivalent of the cyanoacetamide.

13. The process of Claim 1, wherein the alcohol is used in excess to either or both of the cyanoacetamide and the mineral acid.

14. The process of Claim 1, wherein the cyanoacetate is formed in a yield of 70% or greater, preferably wherein the cyanoacetate is formed in a yield of 90% or greater.

15. The process of Claim 1, wherein the cycanoacetate separated in step (b) is substantially free from the cyanoacetamide, mineral acid, and/or alcohol, and by-products.

## Patentansprüche

1. Verfahren für die Herstellung eines Cyanoacetats, dessen Schritte umfassen:
(a) Inberührungbringen von Cyanoacetamid mit einem Alkohol in der Gegenwart einer Mineralsäure unter geeigneten Bedingungen und für eine Zeit, die ausreicht, um ein Cyanoacetat zu erbringen; und
(b) optional Trennen davon des so ausgebildeten Cyanoacetats,
wobei das Cyanoacetamid ausgewählt ist aus Cyanoacetamid, monosubstituierten Cyanoacetamiden oder disubstituierten Cyanoacetamiden, deren jeweilige Substituenten Alkyl, Alkenyl, Alkinyl oder Aryl sind.

2. Verfahren nach Anspruch 1, wobei das Cyanoacetat ein C₁₋₂₀-Alkylcyanoacetat, ein C₆₋₂₀-Arylcyanoacetat, ein C₇₋₂₀-Alkarylcyanoacetat oder ein C₇₋₂₀-Aralkylcyanoacetat ist, wobei ein beliebiges davon durch eine oder mehrere Hydroxylgruppen oder C₁₋₂₀-Alkylethergruppen substituiert sein kann.

3. Verfahren nach Anspruch 1, wobei das Cyanoacetat ein C₁₋₂₀₋-Alkylcyanoacetat ist, wobei das C₁₋₂₀₋-Alkyl eine oder mehrere Stellen einer Ungesättigtheit enthalten kann und durch ein oder mehrere Heteroatome oder heteroatomhaltige Gruppen substituiert und/oder unterbrochen sein kann oder durch Halogene substituiert oder durch halogenhaltige Gruppen substituiert oder unterbrochen sein kann.

4. Verfahren nach Anspruch 1, wobei das Cyanoacetat ein C₁₋₂₀-Alkylcyanoacetat ist, das ausgewählt ist aus Methylcyanoacetat, Ethylcyanoacetat, Propylcyanoacetaten, Butylcyanoacetaten, Pentylcyanoacetaten, Octylcyanoacetaten, Alkoxyetheralkylcyanoacetaten, Allylcyanoacetaten und Kombinationen davon.

5. Verfahren nach Anspruch 1, wobei das Cyanoacetat ein C₆₋₂₀-Arylcyanoacetat ist, ausgewählt aus Phenylcyanoacetat.

6. Verfahren nach Anspruch 1, wobei das Cyanoacetat ein C₇₋₂₀-Aralkylcyanoacetat ist, ausgewählt aus Phenethylcyanoacetat, Benzylcyanoacetat oder Toluylcyanoacetat.

7. Verfahren nach Anspruch 1, wobei der Alkohol ein Alkylalkohol, ein Arylalkohol, ein Alkarylalkohol oder ein Aralkylalkohol ist.

8. Verfahren nach Anspruch 1, wobei der Alkohol ausgewählt ist aus Methanol, Ethanol, Propanolen, Proparganolen, Butanolen, Pentanolen, Hexanolen, Octanolen, Nonanolen, Oxononanolen, Decanolen, Dodecanolen, Allanol, Cyclohexanol, Tetrahydrofurfurol, Chlorethanol, 2,2,2-Trifluorethanol, Hexafluorisopropanol, Alkoxyetheralkanolen, Dialkylsiloxanolen oder Trialkylsilylalkanolen.

9. Verfahren nach Anspruch 1, wobei der Alkohol ein aromatischer Alkohol ist,
vorzugsweise wobei der Alkohol ausgewählt ist aus Phenol oder Benzylalkohol.

10. Verfahren nach Anspruch 1, wobei die Mineralsäure Schwefelsäure, schweflige Säure, Sulfonsäure, Phosphorsäure, phosphorige Säure, Phosphonsäure, Salzsäure oder Bromwasserstoffsäure ist.

11. Verfahren nach Anspruch 1, wobei die Mineralsäure in einer Menge von 0,5 bis 1,5 Äquivalenten zu 1 Äquivalent des Cyanoacetamids verwendet wird.

12. Verfahren nach Anspruch 1, wobei die Mineralsäure in einer Menge von 0,6 bis 1,2 Äquivalenten zu 1 Äquivalent des Cyanoacetamids verwendet wird.

13. Verfahren nach Anspruch 1, wobei der Alkohol im Überschuss gegenüber dem Cyanoacetamid oder der Mineralsäure oder beidem verwendet wird.

14. Verfahren nach Anspruch 1, wobei das Cyanoacetat in einer Ergiebigkeit von 70 % oder mehr ausgebildet wird, vorzugsweise wobei das Cyanoacetat in einer Ergiebigkeit von 90 % oder mehr ausgebildet wird.

15. Verfahren nach Anspruch 1, wobei das Cyanoacetat, das in Schritt (b) abgetrennt wird, im Wesentlichen frei von dem Cyanoacetamid, der Mineralsäure und/oder dem Alkohol und Nebenprodukten ist.

## Revendications

1. Procédé pour la préparation d'un cyanoacétate, dont les étapes comprennent :
(a) la mise en contact d'un cyanoacétamide avec un alcool, en présence d'un acide minéral, dans des conditions appropriées et pendant une durée suffisante pour produire un cyanoacétate ; et,
(b) éventuellement, la séparation du cyanoacétamide ainsi formé,
dans lequel ledit cyanoacétamide est choisi parmi le cyanoacétamide, les cyanoacétamides monosubstitués ou les cyanoacétamides disubstitués dont les substituants éventuels sont alkyle, alcényle, alcynyle ou aryle.

2. Procédé selon la revendication 1, dans lequel le cyanoacétate est un cyanoacétate d'alkyle en C₁₋₂₀, un cyanoacétate d'aryle en C₆₋₂₀, un cyanoacétate d'alcaryle en C₇₋₂₀ ou un cyanoacétate d'aralkyle en C₇₋₂₀, chacun pouvant être substitué par un ou plusieurs groupes hydroxyles ou groupes éther d'alkyle en C₁₋₂₀.

3. Procédé selon la revendication 1, dans lequel le cyanoacétate est un cyanoacétate d'alkyle en C₁₋₂₀, dans lequel l'alkyle en C₁₋₂₀ peut contenir un ou plusieurs points d'insaturation et peut être substitué et/ou interrompu par un ou plusieurs hétéroatomes ou groupes contenant des hétéroatomes, ou substitué par des halogènes ou substitué ou interrompu par des groupes contenant des halogènes.

4. Procédé selon la revendication 1, dans lequel le cyanoacétate est un cyanoacétate d'alkyle en C₁₋₂₀ choisi parmi le cyanoacétate de méthyle, le cyanoacétate d'éthyle, les cyanoacétates de propyle, les cyanoacétates de butyle, les cyanoacétates de pentyle, les cyanoacétates d'octyle, les cyanoacétates d'alcoxyétheralkyle, le cyanoacétate d'allyle, et leurs combinaisons.

5. Procédé selon la revendication 1, dans lequel le cyanoacétate est un cyanoacétate d'aryle en C₆₋₂₀ choisi parmi le cyanoacétate de phényle.

6. Procédé selon la revendication 1, dans lequel le cyanoacétate est un cyanoacétate d'aralkyle en C₇₋₂₀ choisi parmi le cyanoacétate de phénéthyle, le cyanoacétate de benzyle ou le cyanoacétate de toluyle.

7. Procédé selon la revendication 1, dans lequel l'alcool est un alcool d'alkyle, un alcool d'aryle, un alcool d'alcaryle ou un alcool d'aralkyle.

8. Procédé selon la revendication 1, dans lequel l'alcool est choisi parmi le méthanol, l'éthanol, les propanols, les proparganols, les butanols, les pentanols, les hexanols, les octanols, les nonanols, les oxononanols, les décanols, dodécanols, allanol, cyclohexanol, tétrahydrofurfurol, chloroéthanol, 2,2,2- trifluoroéthanol, hexafluoroisopropanol, alcoxy-éther-alcanols, dialkyl siloxanols ou trialkylsilylalcanols.

9. Procédé selon la revendication 1, dans lequel l'alcool est un alcool aromatique,
de préférence dans lequel l'alcool est choisi parmi le phénol ou l'alcool de benzyle.

10. Procédé selon la revendication 1, dans lequel l'acide minéral est de l'acide sulfurique, de l'acide sulfureux, de l'acide sulfonique, de l'acide phosphorique, de l'acide phosphoreux, de l'acide phosphonique, de l'acide chlorhydrique ou de l'acide bromhydrique.

11. Procédé selon la revendication 1, dans lequel l'acide minéral est utilisé en une quantité de 0,5 à 1,5 équivalent pour 1 équivalent du cyanoacétamide.

12. Procédé selon la revendication 1, dans lequel l'acide minéral est utilisé en une quantité de 0,6 à 1,2 équivalent pour 1 équivalent du cyanoacétamide.

13. Procédé selon la revendication 1, dans lequel l'alcool est utilisé en excès par rapport au cyanoacétamide et à l'acide minéral ou aux deux.

14. Procédé selon la revendication 1, dans lequel le cyanoacétate est formé dans une proportion de 70 % ou plus, de préférence dans lequel le cyanoacétate est formé dans une proportion de 90 % ou plus.

15. Procédé selon la revendication 1, dans lequel le cyanoacétate séparé à l'étape (b) est sensiblement exempt du cyanoacétamide, d'acide minéral, et/ou d'alcool et de produits secondaires.
